# EUROPÄISCHE PATENTSCHRIFT

(11) **EP 3 712 579 B1**
(45) Veröffentlichungstag und Bekanntmachung des Hinweises auf die Patenterteilung: **29.12.2021**
(21) Anmeldenummer: 19163506.9
(22) Anmeldetag: 18.03.2019
(51) Int. Cl.: G01J 3/46, G06F 17/10

(54) **VERFAHREN ZUR GENERIERUNG EINER ZUSAMMENSETZUNG FÜR FARBEN, LACKE, DRUCKFARBEN, ANREIBEHARZE, PIGMENTKONZENTRATE ODER SONSTIGE BESCHICHTUNGSSTOFFE**
METHOD FOR GENERATING A COMPOSITION FOR PAINTS, VARNISHES, INKS, GRINDING RESINS, PIGMENT CONCENTRATES OR OTHER COATINGS
PROCÉDÉ DE GÉNÉRATION D'UNE COMPOSITION POUR PEINTURES, LAQUES, ENCRES D'IMPRIMERIE, RÉSINES DE BROYAGE, CONCENTRÉS DE PIGMENT OU AUTRES MATIÈRES DE REVÊTEMENT

(43) Veröffentlichungstag der Anmeldung: 23.09.2020
(73) Patentinhaber: Evonik Operations GmbH, 45128 Essen (DE)
(72) Erfinder: KROEHL, Oliver, 50933 Köln (DE); BLANDA, Gaetano, 45721 Haltern am See (DE); SILBER, Stefan, 47804 Krefeld (DE); BITTORF, Sandra, 44805 Bochum (DE); ISKEN, Phillip, 48341 Altenberge (DE); HUSEN, Inga, 44137 Dortmund (DE); SCHÖNEBERG, Ulf, 12101 Berlin (DE)
(74) Vertreter: Richardt Patentanwälte PartG mbB

(56) Entgegenhaltungen:
- EP-A1- 0 767 362
- EP-A1- 1 512 950
- EP-A1- 2 887 275
- WO-A1-2014/076619
- US-A1- 2004 122 648

## Beschreibung

Die Erfindung betrifft ein Verfahren zur Generierung einer Zusammensetzung für Farben, Lacke, Druckfarben, Anreibeharze, Pigmentkonzentrate oder sonstige Beschichtungsstoffe, wobei die Zusammensetzung Feststoffe und Dispergiermittel umfasst.

### Stand der Technik

Zusammensetzungen für Farben-, Lacke-, Druckfarbenzusammensetzungen, Anreibeharze, Pigmentkonzentrate und sonstige Beschichtungsstoffe, sind komplexe Mischungen von Rohstoffen. Übliche Zusammensetzungen oder Rezepturen bzw. Formulierungen für Farben, Lacke, Druckfarben, Anreibeharze, Pigmentkonzentrate oder sonstige Beschichtungsstoffe enthalten etwa 20 Rohstoffe im Folgenden auch "Komponenten" genannt. Diese Zusammensetzungen bestehen beispielsweise aus Rohstoffen, die ausgewählt sind aus Feststoffen, wie z.B. Pigmenten und/oder Füllstoffen, Bindemitteln, Lösemitteln, Harzen, Härtern und verschiedenen Additiven, wie Verdicker, Dispergiermittel, Benetzer, Haftvermittler, Entschäumer, Oberflächenmodifizierungsmittel, Verlaufmittel, katalytisch wirksame Additive, wie z.B. Trockenstoffe und Katalysatoren, und speziell wirksamen Additive, wie z.B. Biozide, Photoinitiatoren und Korrosionsinhibitoren.

Bisher werden neue Zusammensetzungen oder Formulierungen mit bestimmten, gewünschten Eigenschaften anhand von Erfahrungswerten spezifiziert und danach chemisch synthetisiert und getestet. Die Komposition einer neuen Zusammensetzung oder Formulierung, die bestimmte Erwartungen an deren chemischen, physikalischen, optischen, haptischen und sonstigen messtechnisch erfassbaren Eigenschaften erfüllt, ist aufgrund der Komplexität der Wechselwirkungen auch für einen Fachmann kaum vorhersehbar. Durch die Mannigfaltigkeit der Wechselwirkungen der Rohstoffe untereinander und damit einhergehend einer Vielzahl von (teilweise manuell ausgeführten) Versuchen und Fehlversuchen ist dieses Vorgehen sowohl zeit- als auch kostenintensiv

Für die Dispergierung von Feststoffen (z.B. Pigmenten, Füllstoffen oder Farbstoffen) in flüssigen Medien bedient man sich in der Regel Dispergiermitteln (auch Dispergieradditive genannt), um eine effektive Dispergierung der Feststoffe zu erreichen, die zur Dispergierung benötigten mechanischen Scherkräfte zu reduzieren und gleichzeitig möglichst hohe Füllgrade zu realisieren. Die Dispergiermittel unterstützen das Aufbrechen von Agglomeraten, benetzen und/oder belegen als oberflächenaktive Materialien die Oberfläche der zu dispergierenden Feststoffe bzw. Partikel und stabilisieren diese gegen eine unerwünschte Reagglomeration.

Dispergiermittel werden auch bei der Herstellung von Farben, Lacken, Druckfarben, Anreibeharzen, Pigmentkonzentraten und sonstigen Beschichtungsstoffen eingesetzt, da durch die Dispergiermittel die Einarbeitung von Feststoffen, wie zum Beispiel Pigmenten, Farbstoffen und Füllstoffen, die als wichtige Zusammensetzungsbestandteile das optische Erscheinungsbild und die physikalisch-chemischen Eigenschaften von derartigen Systemen wesentlich bestimmen, erleichtert wird. Für eine optimale Ausnutzung müssen diese Feststoffe zum einen gleichmäßig in den Formulierungen verteilt werden, zum anderen muss die einmal erreichte Verteilung stabilisiert werden.

Eine Vielzahl verschiedener Substanzen findet heute Verwendung als Dispergiermittel für Feststoffe. Neben sehr einfachen, niedermolekularen Verbindungen, wie z.B. Lecithin, Fettsäuren und deren Salze und Alkylphenolethoxylate, werden auch komplexere hochmolekulare Strukturen als Dispergiermittel eingesetzt. Hier sind es speziell amino- und amidofunktionelle Systeme, die breite Verwendung finden.

US-A-4 224 212, EP-B-0 208 041, WO-A-00/24503 und WO-A-01/21298 beschreiben zum Beispiel Dispergiermittel auf der Basis von Polyester-modifizierten Polyaminen. DE-B-197 32 251 beschreibt Polyaminsalze und deren Einsatz als Dispergiermittel für Pigmente und Füllstoffe.

EP 1 512 950 A1 ist auf ein approximatives Zusammensetzungssuchsystem gerichtet, welches die Performance von Zusammensetzungen auswertet. EP 2 887 275 A1, WO 2014/076619 A1, EP 0 767 362 A1, US 2004/122648 A1 sind auf Color Matching Systeme gerichtet, welche Zusammensetzungen mit bestimmten Charakteristika suchen.

### Zusammenfassung

Es ist die Aufgabe der vorliegenden Erfindung, ein Verfahren bereitzustellen, durch welches die Entwicklung einer neuen Zusammensetzung oder die Entwicklung einer Reformulierung zeitsparender und kostengünstiger erreicht wird.

Die Aufgabe wird durch das Verfahren zur Generierung einer Zusammensetzung für Farben, Lacke, Druckfarben, Anreibeharze, Pigmentkonzentrate oder sonstige Beschichtungsstoffe, wobei die Zusammensetzung Feststoffe und Dispergiermittel umfasst, gemäß Anspruch 1 sowie ein entsprechendes Computersystem und Computerprogrammprodukt gelöst. Ausführungsformen der Erfindung sind in den abhängigen Ansprüchen angegeben. Ausführungsformen der vorliegenden Erfindung können frei miteinander kombiniert werden, wenn sie sich nicht gegenseitig ausschließen.

In einem Aspekt betrifft die Erfindung ein Verfahren zur Generierung einer Zusammensetzung für Farben, Lacke, Druckfarben, Anreibeharze, Pigmentkonzentrate oder sonstige Beschichtungsstoffe, wobei die Zusammensetzung durch ein Computersystem generiert wird. Das Computersystem hat Zugriff auf eine Datenbank, in der bekannte Zusammensetzungen und/oder Formulierungen gespeichert sind.

Unter einer "Zusammensetzung" wird hier eine Spezifikation eines chemischen Erzeugnisses verstanden, welche zumindest die Art der Rohstoffe ("Komponenten") spezifiziert, aus denen das chemische Erzeugnis gebildet wird.

Unter einer "Formulierung" wird hier eine Zusammensetzung verstanden, welche neben der Angabe der Komponenten zusätzlich auch Mengen- oder Konzentrationsangaben für die jeweiligen Komponenten umfasst.

Wenn im Weiteren von Zusammensetzungen die Rede ist, kann das je nach Ausführungsform auch bedeuten, dass eine Formulierung vorliegt.

Für die bekannten Zusammensetzungen, die jeweils Feststoff und Dispergiermittel umfassen, sind in der Datenbank jeweils Messpunkte für einen Feststoffanteil und eine Dispergiermittelkonzentration bzw. Dispergiermittelanteil gespeichert.

Für jede bekannte Zusammensetzung können beispielsweise mehrere Messpunkte in der Datenbank gespeichert sein. Beispielsweise können mehrere Messpunkte für einen (festgelegten) Feststoffanteil mit variierendem Dispergiermittelanteil oder für einen (festgelegten) Dispergiermittelanteil mit variierendem Feststoffanteil gespeichert sein. Es ist zu dem Verfahren gehörig, dass für jeden Messpunkt einer jeden Zusammensetzung, beispielsweise für jede getestete Dispergiermittelkonzentration mit einem zugeordneten Feststoffanteil, jeweils mindestens eine rheologische Eigenschaft und mindestens eine koloristische Eigenschaft in der Datenbank gespeichert sind.

Die rheologische Eigenschaft kann beispielsweise die Viskosität der betreffenden Zusammensetzung sein. Die koloristische Eigenschaft kann beispielsweise die Farbstärke der betreffenden Zusammensetzung sein.

Die Zusammensetzungen können Farben, Lacke, Injektink, Pigmentpasten, Pigmentkonzentrate, Anreibeharze oder sonstige Beschichtungsstoffe, wie beispielsweise Vormischungen bestimmter Rohstoffe (als Convenience-Produkt), Dispersionen von Feststoffen, Suspensionen, wässrige und lösemittelhaltige Zusammensetzungen, 100%-Zusammensetzungen (enthalten kein Wasser und kein Lösemittel) oder UV-basierte Farb- und Lacksysteme, sein.

Dispergiermittel bzw. grenzflächenaktive Verbindungen im Sinne der vorliegenden Erfindung können chemische amphiphile, ionische, nichtionische oder niedermolekulare Verbindungen und/oder hochmolekulare Verbindungen sein.

Die Dispergiermittel bzw. grenzflächenaktiven Verbindungen können polyethermodifizierte Fettsäuren, polyethermodifizierte Fettsäureamidamine, polyethermodifierte Amin Derivate, Jeffamin Derivate, polyethermodifizierte Öle, Fette und deren Derivate, phosporylierte Polyether-Derivate, insbesondere auf Fettalkohol Basis, Maleinatharze oder polyethermodifizierte Styrol Maleinsäure Copolymere.

Bei Maleinatharzen handelt es sich um die Diels-Alder-Addukte von Kolophonium mit Malein- bzw. Fumarsäure, welche noch ganz oder teilweise mit mehrwertigen

Alkoholen verestert werden können. So können eine sehr große Bandbreite an Hartharzen mit unterschiedlichen Schmelzpunkten, Funktionalitäten sowie dadurch bedingten Löslichkeiten hergestellt werden.

Die Dispergiermittel bzw. grenzflächenaktiven Verbindungen können auch Polyester sein, die mit pigmentaffinen Gruppen modifiziert sind.

Die Dispergiermittel bzw. grenzflächenaktiven Verbindungen können auch nichtionische ethoxylierte Zuckertenside sein, wie beispielsweise Polyoxyethylen-sorbitan-monolaurat, Polyoxyethylen-sorbitan-monopalmitat, Polyoxyethylen-sorbitan-monostearat, Polyoxyethylen-sorbitan-monooleat und Polyoxyethylen-sorbitan-tristearat.

Alternativ können die Dispergiermittel bzw. grenzflächenaktiven Verbindungen handelsübliche Fettalkoholethoxylate, ausgewählt aus der Gruppe der Polyalkylenglycolether sein.

Es kann vorgesehen sein, dass die Dispergiermittel bzw. grenzflächenaktiven Verbindungen eine oder mehrere Funktionalitäten aufweisen, die eine Affinität für eine Pigmentoberfläche aufweisen.

Ein Feststoff im Sinne der vorliegenden Erfindung kann prinzipiell jedes feste organische oder anorganische Material oder Ruß sein.

Beispiele solcher Feststoffe sind Pigmente, Füllstoffe, Farbstoffe, optische Aufheller, keramische Materialien, magnetische Materialien, nanodisperse Feststoffe, Metalle, Biozide, Agrochemikalien und Pharmaka.

Ein bevorzugter Feststoff ist ein Pigment aus irgendeiner der anerkannten Klassen von Pigmenten, die beispielsweise in Third Edition of the Colour Index (1971) und nachfolgenden Neubearbeitungen und Ergänzungen dazu unter dem Kapitel mit der Überschrift "Pigmente" beschrieben werden.

Vorzugsweise handelt es sich bei den Pigmenten um organische, anorganische Pigmente oder Rußpigmente.

Als anorganische Pigmente können beispielsweise Eisenoxide, Chromoxide oder Titanoxide genannt werden.

Geeignete organische Pigmente sind beispielsweise Azopigmente, Metallkomplex-Pigmente, anthrachinoide Pigmente, Phthalocyaninpigmente, polycyclische Pigmente, insbesondere solche der Thioindigo-, Chinacridon-, Dioxazin-, Pyrrolopyrrol-, Napthalenetetracarbonsäure-, Perylen-, Isoamidolin(on)-, Flavanthron-, Pyranthron- oder Isoviolanthron-Reihe.

Als Ruße können Gasruße, Flammruße oder Furnaceruße eingesetzt werden. Diese Ruße können zusätzlich nachoxidiert und/oder verperlt sein.

Weitere bevorzugte Feststoffe sind Streckmittel und Füllstoffe, wie Talk, Kaolin, Siliciumdioxid, Baryt und Kreide; teilchenförmige keramische Materialien, wie Aluminiumoxid, Siliciumdioxid, Zirkoniumdioxid, Titandioxid, Siliciumnitrid, Bornitrid, Siliciumcarbid, Borcarbid, gemischte Silicium-Aluminium-Nitride und Metalltitanate, teilchenförmige magnetische Materialien, wie die magnetischen Oxide von Übergangsmetallen, insbesondere Eisen und Chrom, z.B. Gamma-Fe₂O₃, Fe₃O₄ und mit Kobalt dotierte Eisenoxide, Calciumoxid, Ferrite, insbesondere Bariumferrite und Metallteilchen, insbesondere metallisches Eisen, Nickel, Kobalt und Legierungen davon, und Agrochemikalien, wie die Fungizide Flutriafen, Carbendazim, Chlorthalonil und Mancozeb.

Wie zuvor ausgeführt, umfasst die Zusammensetzung für Farben, Lacke, Druckfarben, Anreibeharze, Pigmentkonzentrate oder sonstige Beschichtungsstoffe Feststoffe und Dispergiermittel.

Eine geeignete Kombination von Feststoff, insbesondere einem Pigment, und Dispergiermittel kann eine optimierte Benetzung der Feststoffoberflächen mit den Dispergiermitteln eine optimierte Verteilung und Stabilisierung der Feststoffe, hier insbesondere der Pigmente, in der Matrix der Zusammensetzung bewirken.

Da der Benetzungsgrad der Pigmente nicht immer bekannt oder nicht direkt messbar ist, werden als Indikatoren die rheologischen Eigenschaften, wie z. B. die Viskosität, und die koloristischen Eigenschaften, wie etwa die Farbstärke, herangezogen.

Durch die Wahl des Dispergiermittels und deren Konzentration können die Einsatzmenge von beispielsweise. hochpreisigen Feststoffen, wie manche Pigmenten, reduziert werden, ohne Einbußen bei der Farbstärke in Kauf nehmen zu müssen. Weiterhin wird das Viskositätsverhalten von flüssigen Medien, die Feststoffe, wie z.B. Pigmente, enthalten, wesentlich durch das verwendete Dispergiermittel mitbestimmt. Hier sind vor allem Dispergiermittel gefragt, die eine möglichst geringe Viskosität in den flüssigen Farben und Lacken hervorrufen und bei Lagerung diese auch beibehalten.

Das Verfahren gemäß Anspruch 1 wird durchgeführt, indem zunächst eine Eingabe einer Spezifikation einer gewünschten Kombination einer ersten Komponente und einer zweiten Komponente einer Zusammensetzung über ein Nutzerinterface des Computersystems erfolgt. Das Computersystem hat Zugriff auf eine Datenbank.

Eine Datenbank im Sinne der vorliegenden Erfindung kann jeglicher Speicher sein, in dem Daten, insbesondere strukturierte Daten, gespeichert werden können. Bei der Datenbank kann es sich um eine Datenbank handeln, die eine Textdatei, eine Spreadsheet-Datei, ein Verzeichnis in einem Verzeichnisbaum, oder eine Datenbank eines Datenbankmanagementsystem (DBMS), z.B. MySQL oder PostgreSQL, ist.

Für das Verfahren ist vorgesehen, dass sich bei den Komponenten, nämlich der ersten Komponente und der zweiten Komponente, um einen Feststoff und ein Dispergiermittel handelt.

Für den Fall, dass die erste Komponente der Feststoff ist, ist die zweite Komponente das Dispergiermittel. Weiterhin ist für den Fall, dass die erste Komponente das Dispergiermittel ist, die zweite Komponente der Feststoff.

Zum besseren Nachvollziehen der Verfahrensschritte wird nachfolgend die erste Komponente als erste Komponente (A) und die zweite Komponente als zweite Komponente (B) bezeichnet.

Nach der Eingabe einer Spezifikation einer gewünschten Kombination der ersten Komponente (A) und der zweiten Komponente (B) wird eine Datenbankabfrage mit der gewünschten Kombination der ersten Komponente(A) und der zweiten Komponente (B) als Suchkriterium durchgeführt.

Für den Fall, dass das die Datenbankabfrage eine bekannte Zusammensetzung liefert, die das Suchkriterium - erste Komponente (A) und zweite Komponente (B) - erfüllt, wird die bekannte Zusammensetzung - mit der ersten Komponente(A) und der zweiten Komponente (B) - ausgegeben. Somit sind für die bekannte Zusammensetzung bereits Messpunkte für einen Feststoffanteil und eine Dispergiermittelkonzentration gespeichert, wobei für jeden Messpunkt die Viskosität und die Farbstärke gespeichert sind.

Für den Fall, dass das die Datenbankabfrage keine bekannte Zusammensetzung liefert, die das Suchkriterium - erste Komponente (A) und zweite Komponente (B) - erfüllt, wird eine weitere Datenbankabfrage einer Zusammensetzung mit der ersten Komponente (A) und der zweiten Komponente (B) als Suchkriterium durchgeführt, um eine Kandidatenzusammensetzung aufzufinden, die eine der beiden Komponenten (erste Komponente (A) oder zweite Komponente (B)) sowie anstelle der anderen Komponente (zweite Komponente (B) oder erste Komponente (A)) eine Austauschkomponente umfasst.

Zum einfacheren Nachvollziehen der Erfindung wird beispielshaft die eine der beiden Komponenten, die in der Kandidatenzusammensetzung vorhanden ist, als zweite Komponente (B) und die andere Komponente, die in der Kandidatenzusammensetzung nicht vorhanden ist, als erste Komponente (A) bezeichnet. Es sei angemerkt, dass die eine der beiden Komponenten ebenso als erste Komponente (A) und die andere Komponente ebenso als zweite Komponente (B) bezeichnet werden könnte, jedoch zum einfacheren Nachvollziehen der Erfindung in dem Beispiel nicht verwendet wird.

Die Austauschkomponente wird zum einfacheren Nachvollziehen in diesem Beispiel als Austauschkomponente (X) bezeichnet.

Die Kandidatenzusammensetzung umfasst somit in diesem Beispiel die Austauschkomponente (X) und die zweite Komponente (B).

Daraufhin wird eine Datenbankabfrage durchgeführt, um eine erste bekannte Vergleichszusammensetzung aufzufinden, die die andere der beiden Komponenten, in diesem Beispiel die erste Komponente (A), umfasst sowie eine dritte Komponente, die zum einfacheren Nachvollziehen in diesem Beispiel als dritte Komponente (C) bezeichnet wird.

Die erste bekannte Vergleichszusammensetzung umfasst somit in diesem Beispiel die erste Komponente (A) und eine dritte Komponente (C).

Im nächsten Schritt wird eine Datenbankabfrage durchgeführt, um eine zweite bekannte Vergleichszusammensetzung aufzufinden, die anstelle der anderen Komponente, in diesem Beispiel die erste Komponente (A), die Austauschkomponente, die zum einfacheren Nachvollziehen in diesem Beispiel als Austauschkomponente (X) bezeichnet wurde, sowie die dritte Komponente (C) umfasst.

Die zweite bekannte Vergleichszusammensetzung umfasst somit in diesem Beispiel die Austauschkomponente (X) und die dritte Komponente (C).

In einem weiteren Schritt wird die erste und die zweite bekannte Vergleichszusammensetzung durch Prüfung eines Ähnlichkeitskriteriums, beispielsweise ein vordefinierter Schwellenwert, hinsichtlich der in der Datenbank gespeicherten Messpunkte der ersten und der zweiten bekannten Vergleichszusammensetzung verglichen.

Für den Fall, dass die erste und die zweite bekannte Vergleichszusammensetzung als ausreichend ähnlich bewertet werden, wird die Generierung der Zusammensetzung durchgeführt, wobei in der Kandidatenzusammensetzung die Austauschkomponente (X) gegen die andere der beiden Komponenten, in diesem Beispiel die erste Komponente (A), ausgetauscht wird.

Daraufhin erfolgt die Ausgabe der generierten Zusammensetzung.

Ausführungsformen der Erfindung machen sich die überraschende Erkenntnis zunutze, dass hinreichend ähnliche Vergleichszusammensetzungen den Schluss zulassen, dass in der Kandidatenzusammensetzung die nicht gewünschte Austauschkomponente (X) durch die erste Komponente (A) ausgetauscht werden kann, und man hierdurch eine Prognose für eine praktisch nutzbare Zusammensetzung oder Formulierung erhält. Die Zusammensetzung bzw. Formulierung kann anschließend durch Herstellung und Prüfung von einer Hochdurchsatzanlage (High Throughput Environment/HTE-Anlage) validiert werden.

Für eine Ausgestaltung der Erfindung ist vorgesehen, dass für den Fall, dass mehrere Kandidatenzusammensetzung aufgefunden werden, die Schritte der Datenbankabfragen der ersten und der zweiten Vergleichszusammensetzungen, gefolgt von dem Vergleich der Vergleichszusammensetzungen, so oft wiederholt werden bis für wenigstens eine der mehreren Kandidatenzusammensetzungen die Erfüllung des Ähnlichkeitskriteriums festgestellt wird und für diese Kandidatenzusammensetzung die Generierung der Zusammensetzung durchgeführt wird, wobei in dieser Kandidatenzusammensetzung die Austauschkomponente (X) gegen die andere der beiden Komponenten ausgetauscht wird. Dies hat den Vorteil, dass die gewählte Kandidatenzusammensetzung mit dem höchsten Ähnlichkeitsmaß wahrscheinlich eine besonders sichere Prognose für eine praktisch verwendbare Zusammensetzung liefert.

Für eine Ausgestaltung kann vorgesehen sein, dass für den Fall, dass mehrere Kandidatenzusammensetzungen aufgefunden werden, die Schritte der Datenbankabfragen der ersten und der zweiten Vergleichszusammensetzungen, gefolgt von dem Vergleich der Vergleichszusammensetzungen, mehrfach wiederholt werden bis für mehrere Kandidatenzusammensetzungen die Erfüllung des Ähnlichkeitskriteriums festgestellt wird. Hierbei kann als weiterer Schritt vorgesehen sein, dass die Kandidatenzusammensetzungen mit einem Ähnlichkeitsmaß als Sortierungskriterium sortiert werden, wobei die Kandidatenzusammensetzung mit dem höchsten Ähnlichkeitsmaß ausgewählt wird und der Schritt der Generierung der Zusammensetzung durchgeführt wird, wobei in dieser Kandidatenzusammensetzung die Austauschkomponente (X) gegen die andere der beiden Komponenten ausgetauscht wird.

Für alle vorgenannten Ausgestaltungen der Erfindung kann vorgesehen sein, dass in das Ähnlichkeitsmaß ein erster Abstand der Extremwerte der rheologischen Eigenschaft, z.B. der Viskosität, und ein zweiter Abstand der Extremwerte der koloristischen Eigenschaft, z.B. der Farbstärke, jeweils gegenüber unterschiedlichen Verhältnissen von Dispergiermittelkonzentration und Feststoffanteil, zwischen der ersten und der zweiten bekannten Vergleichszusammensetzung eingeht.

Der Abstand kann z.B. die Länge der kürzesten Verbindung der Extremwerte zwischen der ersten und der zweiten bekannten Vergleichszusammensetzung sein. Der Abstand kann ebenso die Länge der geradlinigen Strecke zwischen den Extremwerten im euklidischen Raum, eine Kostenfunktion oder ein mehrdimensionaler Abstand sein, wobei letzterer abhängig ist von der Anzahl der Dimensionen (gemessene Parameter, z.B. Viskositätsmessungen und/oder Farbstärkemessungen über einer variierenden Dispergiermittelkonzentration bei einem festgelegten Feststoffanteil), oder Mehrfachwerte pro Messpunkt.

Für das Ähnlichkeitsmaß kann weiterhin vorgesehen sein, dass für die in der Datenbank gespeicherten Zusammensetzungen Messpunkte unterschiedlicher Lagerungszeiträume gespeichert sind, wobei der Schritt des Vergleichs der ersten und der zweiten Vergleichszusammensetzung für Messpunkte verschiedener Lagerungszeiträume durchgeführt wird, um für jeden der Lagerungszeiträume die ersten und zweiten Abstände zu ermitteln.

Um die Inhomogenität bzw. die Absetzungsneigung der Feststoffe in der Matrix der Zusammensetzung zu testen, werden die Zusammensetzungen nach einem oder mehreren Lagerungszeiträumen (beispielsweise nach zwei Wochen Lagerung bei 50°C und nach vier Wochen Lagerung bei 50°C) erneut gemessen, um eine Aussage über etwaige ungenügende Stabilisierung, Inhomogenität oder Absetzverhalten der Feststoffe in der Zusammensetzung zu treffen.

Weiterhin kann hierbei vorgesehen sein, dass sich das Ähnlichkeitsmaß aus einer Kombination der ersten und zweiten Abstände der Extremwerte ergibt.

Die jeweils beiden Abstände zwischen den Vergleichszusammensetzungen können somit beispielsweise pro Dimension (gemessener Parameter, z.B. Viskosität oder Farbstärke über z.B. variierender Dispergiermittelkonzentration bei einem festgelegten Feststoffanteil oder über z.B. variierendem Feststoffanteil bei einer festgelegten Dispergiermittelkonzentration) miteinander summiert werden oder insgesamt aufsummiert werden. Sollte die Kombination der Abstände einen vorgegebenen Schwellenwert unter- oder überschreiten, kann vorgesehen sein, dass das Ähnlichkeitsmaß als erfüllt gilt.

Als weiterer Schritt kann vorgesehen sein, dass eine Eingabe einer Spezifikation zumindest einer der Dimensionen der Messpunkte erfolgt, wobei die ausgewählte Dimension in der Kombination übergewichtet wird. Die ausgewählte Dimension kann beispielsweise die Viskosität oder die Farbstärke sein.

Alternativ kann vorgesehen sein, dass ein weiterer Schritt vorgesehen ist, wobei eine Eingabe der Spezifikation für einen Wertebereich der Messpunkte vorgenommen wird, wobei in dem Schritt des Vergleichs der ersten und der zweiten bekannten Vergleichszusammensetzung durch Prüfung eines Ähnlichkeitskriteriums hinsichtlich der in der Datenbank gespeicherten Messpunkte der ersten und der zweiten bekannten Vergleichszusammensetzung vorgenommen wird und die Messpunkte nur in dem spezifizierten Wertebereich ausgewertet werden. Die Eingabe kann beispielsweise ein Kundenwunsch sein, beispielsweise ein gewünschter Farbstärkebereich, ein gewünschter Lagerstabilitätsbereich oder eine gewünschte Viskosität.

Alternativ kann vorgesehen sein, dass das Ähnlichkeitsmaß dem Vergleich der Verläufe der Messpunkte entspricht, wobei der Vergleich durch die Methode der kleinsten Quadrate, der Quadrate der Differenzen, eine Varianzanalyse, einen Stringvergleich von Messpunktbereichen oder eine Korrelation durchgeführt wird.

Die jeweiligen Verläufe der Messpunkte der Vergleichszusammensetzungen können als zwei Informationsmengen aufgefasst werden, deren Ähnlichkeit beispielsweise mit Hilfe eines fuzzy retrieval Ansatzes bewertet werden kann, vgl. beispielsweise S. Miyamoto (Two approaches for information retrieval through fuzzy associations, IEEE Trans. Syst. Man Cybernet. SMC-19 123-30 - 1990b Fuzzy Sets in Information Retrieval and Cluster Analysis, 1989, Dordrecht: Kluwer).

Für alle vorgenannten Ausführungsformen des Verfahrens kann vorgesehen sein, dass die erste bekannte Vergleichszusammensetzung neben der anderen (A) der beiden Komponenten und der dritten Komponente (C) wenigstens eine weitere Komponente (z.B. D, D+E, D+E+F, ...) umfasst und die zweite bekannte Vergleichszusammensetzung neben der dritten Komponenten (C) und der Austauschkomponente (X), ein (D), mehrere (D+E; D+E+F) oder alle (D+E+F, ...) der weiteren Komponenten umfasst.

Es kann vorgesehen sein, dass die wenigstens eine weitere Komponente ausgewählt ist aus der Gruppe bestehend aus Pigmenten, Füllstoffen, Farbstoffen, optischen Aufhellern, keramischen Materialien, magnetischen Materialien, nanodispersen Feststoffen, Metallen, Bioziden, Agrochemikalien, Pharmaka, Bindemitteln, Lösemitteln, Netzmitteln, Verlaufshilfsmitteln, Härtern und Entschäumern.

Weiter kann vorgesehen sein, dass der Feststoff ein anorganisches Pigment, ein organisches Pigment oder ein Rußpigment ist.

Das Dispergiermittel kann eine chemisch amphiphile, ionische, nichtionische oder niedermolekulare Verbindung und/oder hochmolekulare Verbindung sein.

Für alle vorgenannten Ausführungsformen des Verfahrens kann weiterhin vorgesehen sein, dass für den Fall, dass die eine der beiden Komponenten der Kandidatenzusammensetzung der Feststoff ist, der Feststoff ein anorganisches Pigment, ein organisches Pigment oder ein Rußpigment ist, die dritte Komponente (C) entsprechend ebenfalls ein anorganisches Pigment, ein organisches Pigment oder ein Rußpigment ist, und für den Fall, dass die eine der beiden Komponenten der Kandidatenzusammensetzung das Dispergiermittel ist, das Dispergiermittel eine chemisch amphiphile, ionische, nichtionische oder niedermolekulare Verbindung und/oder hochmolekulare Verbindung ist, die dritte Komponente (C) entsprechend ebenfalls eine chemisch amphiphile, ionische, nichtionische oder niedermolekulare Verbindung und/oder hochmolekulare Verbindung ist.

Somit sind die eine der beiden Komponenten und die dritte Komponente ähnlich, beispielsweise in ihren Eigenschaften, wie Farbe, Größe, Struktur, Gewicht, Ladung usw..

Es kann vorgesehen sein, dass die in der Datenbank gespeicherten Messpunkte der ersten und der zweiten bekannten Vergleichszusammensetzung oder Vergleichsformulierung durch eine vorangegangene Prüfung der rheologischen Eigenschaft, z.B. der Viskosität, und der koloristischen Eigenschaft, z.B. der Farbstärke, gegenüber einer Dispergiermittelkonzentration und einem Feststoffanteil mittels einer Anlage zur Durchführung eines Herstellungsprozesses einer Formulierung für Farben, Lacke, Druckfarben, Anreibeharze, Pigmentkonzentrate oder sonstige Beschichtungsstoffe und Prüfung einer Formulierung für Farben, Lacke, Druckfarben, Anreibeharze, Pigmentkonzentrate oder sonstige Beschichtungsstoffe gewonnen wurden.

Eine geeignete Anlage zur Durchführung eines Herstellungsprozesses einer Formulierung für Farben, Lacke, Druckfarben, Anreibeharze, Pigmentkonzentrate oder sonstige Beschichtungsstoffe und Prüfung einer Formulierung für Farben, Lacke, Druckfarben, Anreibeharze, Pigmentkonzentrate oder sonstige Beschichtungsstoffe ist beispielsweise die Hochdurchsatzanlage (High Throughput Equipments/ HTE-Anlage) von Chemspeed Technologies AG, welche in der WO 2017/072351 A2 beschrieben ist, oder die in der EP 2 420 817 B1 in Abs. [0054] beschriebene Anlage zur Hochdurchsatzforschung (Hochdurchsatzanlage).

Es kann weiterhin vorgesehen sein, dass das Computersystem über eine Kommunikationsschnittstelle mit der Datenbank und/oder einer Anlage zur Durchführung eines Herstellungsprozesses einer Formulierung für Farben, Lacke, Druckfarben, Anreibeharze, Pigmentkonzentrate oder sonstige Beschichtungsstoffe und Prüfung einer Formulierung für Farben, Lacke, Druckfarben, Anreibeharze, Pigmentkonzentrate oder sonstige Beschichtungsstoffe kommuniziert, wobei die Kommunikationsschnittstelle durch USB, Ethernet, WLAN, LAN, Bluetooth oder eine andere Netzwerkschnittstelle realisiert ist.

Die Ausgabe der generierten Zusammensetzung bzw. Formulierung kann auf dem Nutzerinterface des Computersystems erfolgen. Es kann zusätzlich oder alternativ vorgesehen sein, dass für die generierte Zusammensetzung eine Formulierung gespeichert ist oder dass ausgehend von der generierten Zusammensetzung, durch beispielsweise ein Versuchsplanungsprogramm, eine oder mehrere Formulierungen generiert werden, wobei die gespeicherte Formulierung oder die durch das Versuchsplanungsprogramm generierte Formulierung an einen Prozessor ausgegeben wird, wobei der Prozessor eine Anlage zur Durchführung eines Herstellungsprozesses einer Formulierung für Farben, Lacke, Druckfarben, Anreibeharze, Pigmentkonzentrate oder sonstige Beschichtungsstoffe und Prüfung einer Formulierung für Farben, Lacke, Druckfarben, Anreibeharze, Pigmentkonzentrate oder sonstige Beschichtungsstoffe steuert. Die Anlage weist mindestens zwei Bearbeitungsstationen auf, wobei die mindestens zwei Bearbeitungsstationen über ein Transportsystem miteinander verbunden sind, auf dem selbstfahrende Transportvehikel (z.B. ein zur Aufnahme der Formulierung geeignetes Gefäß oder ein Substrat, sogenanntes Panel, auf dem die Formulierung applizierbar ist) zum Transport der Komponenten der Zusammensetzung und/oder der hergestellten Formulierung zwischen den Bearbeitungsstationen verkehren können. Die hergestellte Formulierung kann beispielsweise in einem zur Aufnahme der Formulierung geeigneten Gefäß, insbesondere einem Gefäß aus Kunststoff oder Glas, zwischen den Bearbeitungsstationen transportiert werden. Ebenso kann die hergestellte Formulierung auf einem Substrat bzw. auf einem Panel appliziert werden, wobei das Substrat bzw. Panel mit der applizierten Formulierung zwischen den Bearbeitungsstationen transportiert wird. Das Substrat bzw. Panel kann aus jedem Material bestehen, auf dem die Formulierung durch einen Verwender appliziert werden kann, insbesondere Glas, Plastik, Beton, Holz, Metall, Stein, .... Der Prozessor steuert die Anlage an, die generierte Zusammensetzung bzw. Formulierung herzustellen, wobei in den mindestens zwei Bearbeitungsstationen die Herstellung der generierten Formulierung und eine Prüfung der rheologischen Eigenschaft und der koloristischen Eigenschaft erfolgt. Beispielsweise kann die Prüfung der rheologischen Eigenschaft und der koloristischen Eigenschaft erfolgen. Daraufhin erfolgt eine Ausgabe der Ergebnisse der Prüfung der rheologischen Eigenschaft und der koloristischen Eigenschaft auf dem Nutzerinterface des Computersystems und/oder die Ergebnisse der Prüfung der rheologischen Eigenschaft und der koloristischen Eigenschaft werden in der Datenbank gespeichert. Die in der Datenbank gespeicherten Ergebnisse bzw. Messpunkte der Formulierung können somit für nachfolgende Verfahren verwendet werden.

Ein weiterer Aspekt der Erfindung ist ein Computersystem zur Generierung einer Zusammensetzung für Farben, Lacke, Druckfarben oder sonstige Beschichtungsstoffe, umfassend eine Datenbank und ein Nutzerinterface, wobei das Computersystem zur Durchführung des zuvor beschriebenen Verfahrens konfiguriert ist.

Ein weiterer Aspekt der Erfindung ist ein Computerprogramm, digitales Speichermedium oder Computerprogrammprodukt mit von einem Prozessor ausführbaren Anweisungen, um das zuvor beschriebenen Verfahren durchzuführen.

Ein weiterer Aspekt der Erfindung ist ein System umfassend eine Anlage zur Durchführung eines Herstellungsprozesses einer Formulierung für Farben, Lacke, Druckfarben, Anreibeharze, Pigmentkonzentrate oder sonstige Beschichtungsstoffe und Prüfung einer Formulierung für Farben, Lacke, Druckfarben, Anreibeharze, Pigmentkonzentrate oder sonstige Beschichtungsstoffe, wobei die Anlage mindestens zwei Bearbeitungsstationen aufweist, wobei die mindestens zwei Bearbeitungsstationen über ein Transportsystem miteinander verbunden sind, auf dem selbstfahrende Transportvehikel zum Transport der Komponenten der Zusammensetzung und/oder der hergestellten Formulierung zwischen den Bearbeitungsstationen verkehren können, wobei in den mindestens zwei Bearbeitungsstationen sowohl die Herstellung der Formulierung als auch eine Prüfung einer rheologischen Eigenschaft, z.B. Viskosität, und einer koloristischen Eigenschaft, z.B. Farbstärke, gegenüber einer Dispergiermittelkonzentration und einem Feststoffanteil einer jeden Zusammensetzung erfolgt, und ein Computersystem zur Generierung einer Zusammensetzung für Farben, Lacke, Druckfarben Anreibeharze, Pigmentkonzentrate oder sonstige Beschichtungsstoffe, umfassend eine Datenbank und ein Nutzerinterface, wobei das Computersystem zur Durchführung des zuvor beschriebenen Verfahrens konfiguriert ist.

### Kurzbeschreibung der Zeichnungen

In den folgenden Abbildungen werden Ausführungsformen der Erfindung in exemplarischer Weise näher erläutert:
- Figur 1: zeigt ein Flussdiagramm eines Verfahrens zur Generierung einer Zusammensetzung für Farben, Lacke, Druckfarben, Anreibeharze, Pigmentkonzentrate oder sonstige Beschichtungsstoffe, wobei die Zusammensetzung für Farben, Lacke, Druckfarben, Anreibeharze, Pigmentkonzentrate oder sonstige Beschichtungsstoffe Feststoffe und Dispergiermittel umfasst;
- Figur 2: zeigt ein Blockdiagramm eines Systems mit einem Computersystem, einer Datenbank und einer HTE-Anlage; und
- Figur 3A-3C: zeigt drei Diagramme für Zusammensetzungen, die jeweils Eisenoxidrotpigmente und Dispergiermittel umfassen, wobei für Zusammensetzungen, die dasselbe Dispergiermittel umfassen Messwerte für die Viskosität und die Farbstärke wiedergegen sind.

### Detaillierte Beschreibung

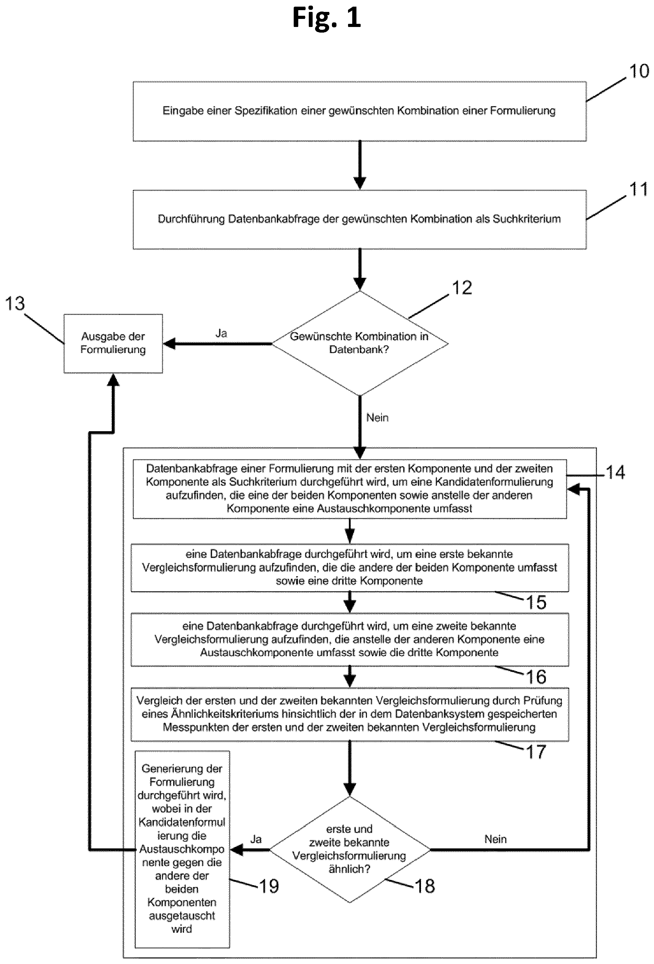

**Figur 1** zeigt ein Flussdiagramm eines Verfahrens zur Generierung einer Zusammensetzung für Farben, Lacke, Druckfarben, Anreibeharze, Pigmentkonzentrate oder sonstige Beschichtungsstoffe.

Das Verfahren kann von einem Computersystem, zum Beispiel einem Kontrollcomputer, einem Notebook, einem Standard-Computer, einem Tablet-Computer oder einem Smartphone ausgeführt werden.

Das Verfahren kann typischerweise im Kontext eines chemischen Labors verwendet werden. In dem Labor befinden sich eine Reihe einzelner Analysegeräte und eine Hochdurchsatz-Anlage (High Throughput Environment/HTE-Anlage), nämlich eine Anlage zur Durchführung eines Herstellungsprozesses einer Formulierung für Farben, Lacke, Druckfarben, Anreibeharze, Pigmentkonzentrate oder sonstige Beschichtungsstoffe und Prüfung einer Formulierung für Farben, Lacke, Druckfarben, Anreibeharze, Pigmentkonzentrate oder sonstige Beschichtungsstoffe. Die HTE Anlage beinhaltet eine Vielzahl von Einheiten und Modulen, die verschiedene chemische oder physikalische Parameter von Substanzen und Substanzmischungen analysieren und messen können und welche eine Vielzahl verschiedener chemische Produkte basierend auf einer von einem Nutzer eingegebenen Rezeptur bzw. Formulierung kombinieren und synthetisieren können.

Das Computersystem hat Zugriff auf eine Datenbank, in welcher bekannte Zusammensetzungen oder Formulierung gespeichert sind und in der für Kombinationen eines Feststoffes und eines Dispergiermittels bekannter Formulierungen jeweils Messpunkte der betreffenden Formulierung für einen Feststoffanteil und eine Dispergiermittelkonzentration gespeichert sind. Für jeden Messpunkt der betreffenden Formulierung sind eine rheologische und eine koloristische Eigenschaft gespeichert.

Die rheologische Eigenschaft kann die Viskosität sein und die koloristische Eigenschaft kann die Farbstärke sein. Sowohl die rheologische Eigenschaft als auch die koloristische Eigenschaft kann mittels der HTE-Anlage ermittelt worden sein.

In einem ersten Schritt 10 macht ein Benutzer, beispielsweise ein Forscher, der neue Zusammensetzungen oder Formulierungen entwickeln möchte, über ein Nutzerinterface 101 des Computersystems 100 eine Eingabe einer Spezifikation einer gewünschten Kombination einer ersten Komponente und einer zweiten Komponente einer Zusammensetzung.

Für das erfindungsgemäße Verfahren ist vorgesehen, dass es sich bei der ersten Komponente und der zweiten Komponente um einen Feststoff und ein Dispergiermittel handelt.

In einem nächsten Schritt 11 erfolgt eine Durchführung einer Datenbankabfrage mit der gewünschten Kombination der ersten Komponente und der zweiten Komponente als Suchkriterium.

Für den Fall, dass die Datenbankabfrage eine bekannte Zusammensetzung oder Formulierung liefert, die das Suchkriterium erfüllt, wird die bekannte Zusammensetzung oder Formulierung in einem weiteren Schritt 13 ausgegeben.

Für den Fall, dass die Datenbankabfrage keine bekannte Zusammensetzung oder Formulierung liefert, die das Suchkriterium erfüllt, wird in einem weiteren Schritt 14 eine weitere Datenbankabfrage einer Zusammensetzung oder Formulierung mit der ersten Komponente und der zweiten Komponente als Suchkriterium durchgeführt, um eine Kandidatenzusammensetzung oder Kandidatenformulierung aufzufinden, die eine der beiden Komponenten sowie anstelle der anderen Komponente eine Austauschkomponente umfasst.

Hierfür wird in einem weiteren Schritt 15 eine Datenbankabfrage durchgeführt, um eine erste bekannte Vergleichszusammensetzung oder Vergleichsformulierung aufzufinden, die die andere der beiden Komponente umfasst sowie eine dritte Komponente.

Zudem wird in einem weiteren Schritt 16 eine Datenbankabfrage durchgeführt, um eine zweite bekannte Vergleichszusammensetzung oder Vergleichsformulierung aufzufinden, die anstelle der anderen Komponente eine Austauschkomponente umfasst sowie die dritte Komponente.

Nach den Schritten 15 und 16 wird in einem weiteren Schritt 17 ein Vergleich der ersten und der zweiten bekannten Vergleichszusammensetzung oder Vergleichsformulierung durch Prüfung eines Ähnlichkeitskriteriums, beispielsweise ein vordefinierter Schwellwert, hinsichtlich der in der Datenbank gespeicherten Messpunkte der ersten und der zweiten bekannten Vergleichszusammensetzung oder Vergleichsformulierung durchgeführt, wobei für den Fall, dass die erste und die zweite bekannte Vergleichszusammensetzung oder Vergleichsformulierung als ausreichend ähnlich bewertet werden, in einem nachfolgenden Schritt 19 die Generierung der Zusammensetzung oder Formulierung durchgeführt wird, wobei in der Kandidatenzusammensetzung oder Kandidatenformulierung die Austauschkomponente gegen die andere der beiden Komponenten ausgetauscht wird, und die die generierte Zusammensetzung oder Formulierung ausgegeben wird, siehe Schritt 13.

Für den Fall, dass die erste und die zweite bekannte Vergleichszusammensetzung oder Vergleichsformulierung nicht als ausreichend ähnlich bewertet werden, werden die Schritte 14 bis 18 erneut durchgeführt.

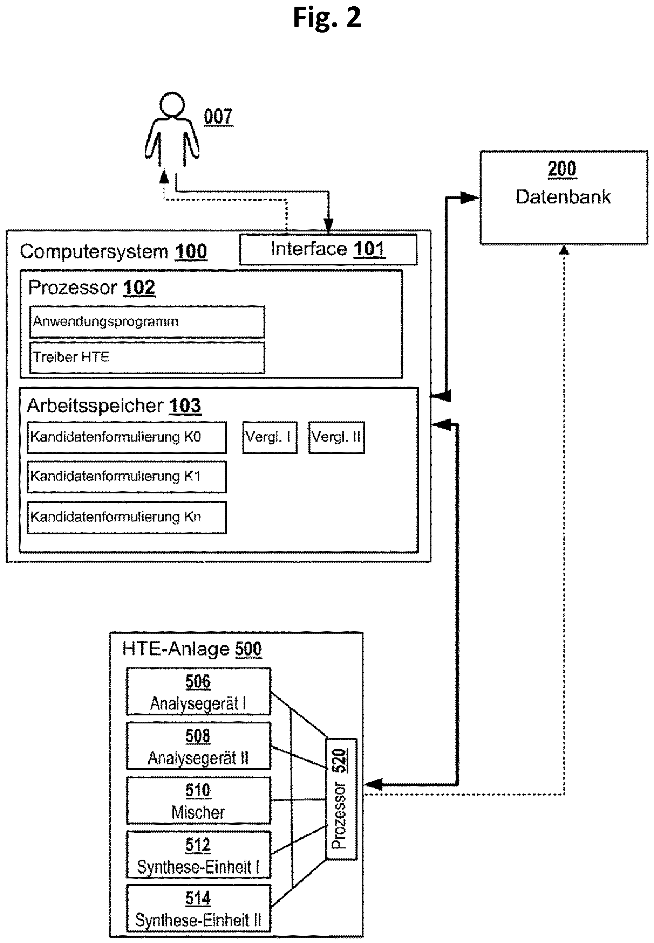

**Figur 2** zeigt ein Blockdiagramm eines Systems mit einem Computersystem 100, einer Datenbank 200 und einer Anlage zur Durchführung eines Herstellungsprozesses einer Zusammensetzung für Farben, Lacke, Druckfarben, Anreibeharze, Pigmentkonzentrate oder sonstige Beschichtungsstoffe und Prüfung einer Zusammensetzung für Farben, Lacke, Druckfarben, Anreibeharze, Pigmentkonzentrate oder sonstige Beschichtungsstoffe (HTE-Anlage) 500.

Die wesentlichen Funktionen der Komponenten des Systems und seiner Komponenten wurden bereits im Hinblick auf Figur 1 beschrieben. Bei dem Computersystem 100 kann es sich beispielsweise um ein Notebook, einen Standard-Computer, einen Tablet-Computer, oder ein Smartphone handeln, das von einem Benutzer 007 bedient wird. Der Benutzer 007 gibt über das Interface 101 des Computersystems beispielsweise die Eingabe einer Spezifikation einer gewünschten Kombination einer Zusammensetzung oder Formulierung ein (siehe auch Schritt 10 in Fig. 1). Die nachfolgend beschriebenen Zusammensetzungen können auch Formulierungen sein.

Das Computersystem 100 hat Zugriff auf eine Datenbank, in der bekannte Zusammensetzungen gespeichert sind und in der für Kombinationen eines Feststoffes und eines Dispergiermittels bekannter Zusammensetzungen jeweils Messpunkte der betreffenden Zusammensetzung für einen Feststoffanteil und eine Dispergiermittelkonzentration gespeichert sind.

Zudem weist das Computersystem 100 einen Prozessor 102 mit einem Anwendungsprogramm sowie einen Treiber zur Steuerung eines Prozessors 520 der HTE-Anlage 500 auf. Wie dargestellt, weist die HTE-Anlage 500 eine Vielzahl von Einheiten und Modulen 506 - 514 auf, die verschiedene chemische oder physikalische Parameter von Substanzen und Substanzmischungen analysieren und messen können. Beispielsweise ist die Einheit 506 ein Analysegerät, dass die Viskosität einstellen und bestimmen kann. Beispielsweise ist die Einheit 508 ein Analysegerät, dass Farbwerte anhand eines integrierten Spektralphotometer (über sog. L-a,b-Wert) ermitteln kann.

Zudem weist das Computersystem 100 einen Arbeitsspeicher 103 auf, in dem die Kandidatenzusammensetzung K0 und weitere Kandidatenzusammensetzungen K1 bis Kn, wobei n ≥ 2 sein kann, und die Vergleichszusammensetzungen Vergl. I, Vergl. II zwischengespeichert werden können.

Wie dargestellt, kann das Computersystem 100 Daten, z.B. Zusammensetzungen und/oder Messwerte und Eigenschaften von Komponenten, sowohl auf der Datenbank 200 speichern, als auch Daten von der Datenbank 200 abrufen. Ebenso kann das Computersystem 100 Daten, z.B. Aufträge, an die HTE-Anlage 500, übermitteln und die Ergebnisse empfangen. Die Ergebnisse können daraufhin an das Datenbanksystem 200 und/oder (über das Interface 101) an den Benutzer 007 weitergegeben werden. Es ist zudem vorgesehen, dass die Ergebnisse, die mittels der HTE-Anlage 500 ermittelt wurden, direkt in der Datenbank 200 eingepflegt werden können.

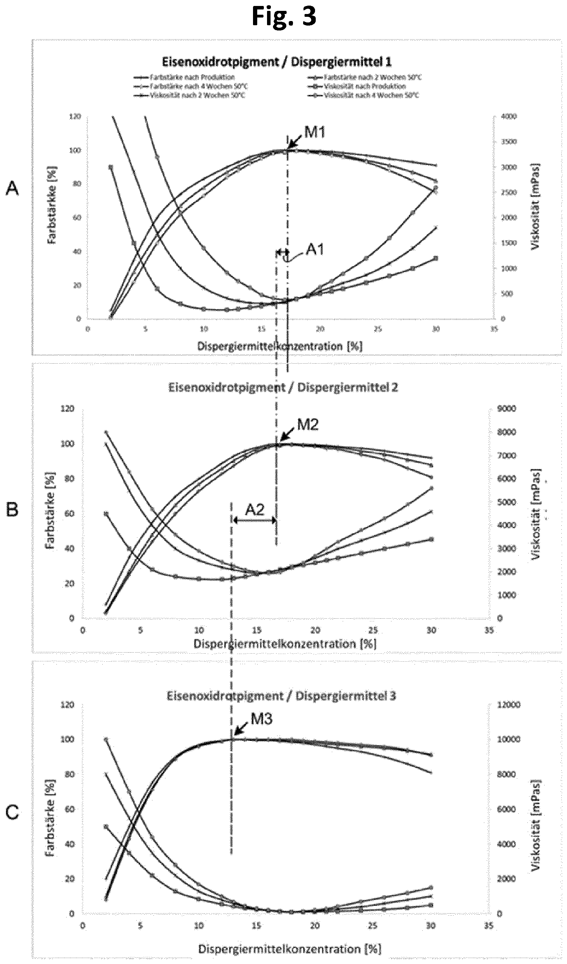

**Figur 3** zeigt drei Diagramme (Fig. 3A, Fig. 3B und Fig. 3C) für Zusammensetzungen, die jeweils Eisenoxidrotpigmente und verschiedene Dispergiermittel umfassen.

In Fig. 3A sind Messwerte für die Farbstärke und die Viskosität von Zusammensetzungen, die ein Dispergiermittel 1 und Eisenoxidrotpigmente umfassen, gegenüber verschiedenen bzw. variierenden Dispergiermittelkonzentrationen bei einem festgelegten Anteil von Eisenoxidrotpigmenten, als Kurvenverläufe dargestellt.

In Fig. 3B sind Messwerte für die Farbstärke und die Viskosität von Zusammensetzungen, die ein Dispergiermittel 2 und Eisenoxidrotpigmente umfassen, gegenüber verschiedenen bzw. variierenden Dispergiermittelkonzentrationen bei einem festgelegten Anteil von Eisenoxidrotpigmenten, als Kurvenverläufe dargestellt.

In Fig. 3C sind Messwerte für die Farbstärke und die Viskosität von Zusammensetzungen, die ein Dispergiermittel 3 und Eisenoxidrotpigmente umfassen, gegenüber verschiedenen bzw. variierenden Dispergiermittelkonzentrationen bei einem festgelegten Anteil von Eisenoxidrotpigmenten, als Kurvenverläufe dargestellt.

Die in den Figuren 3A bis 3C dargestellten Dispergiermittel 1, 2 und 3 sind unterschiedliche wässrige Lösungen eines Copolymers mit pigmentaffinen Gruppen. Die Dispergiermittel 1, 2 und 3 unterscheiden sich untereinander entweder in dem Copolymer, in ihrem Aussehen (klar, trüb oder Farbe), der Säurezahl [mg KOH/g], der Viskosität [mPa·s bei 25 °C], in dem Flammpunkt [°C] und/oder dem Schmelzpunkt (°C). In den Figuren 3A bis 3C sind Messwerte der Viskosität und der Farbstärke für Zusammensetzungen mit variierendem Dispergiermittelanteil, wobei der Dispergiermittelanteil zwischen 1 % und 30 % gegenüber einem festgelegten Anteil von Eisenoxidrotpigmenten (hier 65 %) variiert, dargestellt. Der Rest der Zusammensetzung besteht aus den weiteren Komponenten, hier Additiven, Entschäumern, Wasser und Bioziden.

In den Fig. 3A, 3B und 3C sind die Messwerte für die Farbstärke und die Viskosität von jeder der Zusammensetzungen (mit dem gleichen, jedoch im Anteil variierenden, Dispergiermittel) zudem nach verschiedenen Lagerungszeiträumen dargestellt. Die verschiedenen Lagerungszeiträume sind: i.) nach der Produktion (s. Messpunkte mit dem Kreuzsymbol für die Farbstärke und Messpunkte mit dem Quadratsymbol für die Viskosität), ii.) nach zwei Wochen Lagerung der Zusammensetzung bei 50°C (s. Messpunkte mit dem Dreieckssymbol für die Farbstärke und Messpunkte mit dem Sternsymbol für die Viskosität) und iii.) nach vier Wochen Lagerung der Zusammensetzung bei 50°C (s. Messpunkte mit dem Diamantsymbol für die Farbstärke und Messpunkte mit dem Kreissymbol für die Viskosität).

Die Messwerte für die Farbstärke und die Viskosität wurden durch eine Anlage zur Durchführung eines Herstellungsprozesses einer Formulierung für Farben, Lacke, Druckfarben, Anreibeharze, Pigmentkonzentrate oder sonstige Beschichtungsstoffe und Prüfung einer Formulierung für Farben, Lacke, Druckfarben, Anreibeharze, Pigmentkonzentrate oder sonstige Beschichtungsstoffe (HTE-Anlage von Chemspeed Technologies AG, beschrieben in WO 2017/072351 A2) gemessen und in der Datenbank abgespeichert. Hierbei wurde die Lagerstabilitätsmessungen anhand der Inhomogenität bzw. Absetzungsneigung gemessen, wobei eine Platte/Spindel bei einer Abwärtsbewegung die Normalkraft misst, wodurch Inhomogenitäten, wie Synärese oder Bodensatz, erfasst werden. Die Viskositätsmessungen der Zusammensetzungen, inkl. Viskositätseinstellung durch Verdünnung, wurden ebenfalls durch die HTE-Anlage durchgeführt, indem die Viskosität der Zusammensetzungen in einem Transportvehikel (Gefäß aus Glas) mittels eines Flügelrührers bestimmt wurden. Die Farbwerte der Zusammensetzungen wurden ebenfalls durch die HTE-Anlage, anhand eines integrierten Spektralphotometer (sog. L-a,b-Wert), gemessen.

Für das erfindungsgemäße Verfahren kann beispielsweise vorgesehen sein, dass die erste bekannte Vergleichszusammensetzung (siehe Fig. 1, Schritt 15) eine oder mehrere der Zusammensetzungen aus Fig. 3A ist. Das Eisenoxidrotpigment wäre damit die dritte Komponente und das Dispergiermittel 1 die andere der beiden Komponenten. Weiterhin kann vorgesehen sein, dass die zweite bekannte Vergleichszusammensetzung (siehe Fig. 1, Schritt 16) eine oder mehrere der Zusammensetzungen aus Fig. 3B ist. Das Eisenoxidrotpigment wäre damit die dritte Komponente und das Dispergiermittel 2 die Austauschkomponente.

Daraufhin wird gemäß des erfindungsgemäßen Verfahrens ein Vergleich der ersten und der zweiten bekannten Vergleichszusammensetzung durch Prüfung eines Ähnlichkeitskriteriums, beispielsweise einem zuvor festgelegtem Schwellwert, hinsichtlich der in der Datenbank gespeicherten Messpunkte der ersten und der zweiten bekannten Vergleichszusammensetzung vorgenommen.

In das Ähnlichkeitsmaß kann beispielsweise ein erster Abstand der Extremwerte der Viskosität und/oder ein zweiter Abstand der Extremwerte der Farbstärke, jeweils gegenüber einer Dispergiermittelkonzentration und einem Feststoffanteil, zwischen der ersten (Fig. 3A) und der zweiten (Fig. 3B) bekannten Vergleichszusammensetzung eingehen.

Wie aus der Fig. 3A im Vergleich zu der Figur 3B ersichtlich, ist für die Zusammensetzungen mit einem Dispergiermittelanteil von 16 % bis 19 % die Farbstärke für alle gemessenen Zusammensetzungen in ihrem Maximum (in Fig. 3A durch M1 indiziert und in Fig. 3B durch M3 indiziert), hier bei etwa 100 %. Da die Maxima M1, M2 der Farbstärke zwischen den Figuren 3A und 3B für den Dispergiermittelanteil nicht oder nicht weit voneinander beabstandet sind (siehe Abstand A1), kann vorgesehen sein, dass die erste (Fig. 3A) und die zweite (Fig. 3B) bekannte Vergleichszusammensetzung als ausreichend ähnlich bewertet werden. Hier kann somit beispielsweise der Abstand der Maxima bei ca. 2 % Dispergiermittelkonzentration sein. Beispielsweise werden die Vergleichszusammensetzungen als ähnlich bewertet, wenn das Ähnlichkeitskriterium als Schwellenwert vorgibt, dass der Abstand kleiner als 3% Dispergiermittelkonzentration sein soll. Wie dargestellt, ist ebenfalls aus den Fig. 3A und 3B ersichtlich, dass für die Zusammensetzungen der Figuren 3A und 3B die Schnittpunkte der Lagerungszeitraumverläufe im Vergleich ebenfalls nicht weit (< 3% Dispergiermittelkonzentration) voneinander beabstandet sind. Somit können diese Schnittpunkte ebenfalls als Abstand in das Ähnlichkeitsmaß eingehen, wodurch vorgesehen sein kann, dass die erste (Fig. 3A) und die zweite (Fig. 3B) bekannte Vergleichszusammensetzung als ausreichend ähnlich bewertet werden. Ebenso kann das Ähnlichkeitsmaß dem Vergleich der Verläufe der Messpunkte entsprechen, wobei der Vergleich durch die Methode der kleinsten Quadrate, der Quadrate der Differenzen, eine Varianzanalyse, einen Stringvergleich von Messpunktbereichen oder eine Korrelation durchgeführt wird. Wie hier für Zusammensetzungen mit unterschiedlichen Dispergiermitteln in Fig. 3A und 3B dargestellt, verhalten sich die Verläufe der Messwerte der Zusammensetzungen nach der Produktion sehr ähnlich, daher können sie als ausreichend ähnlich bewertet werden.

Da die erste und die zweite bekannte Vergleichszusammensetzung als ausreichend ähnlich bewertet wurden, kann gemäß des beanspruchten Verfahrens die Generierung der Zusammensetzung durchgeführt werden, wobei in der Kandidatenzusammensetzung die Austauschkomponente (Dispergiermittel 2) gegen die andere der beiden Komponenten (Dispergiermittel 1) ausgetauscht wird, wonach die Ausgabe der generierten Zusammensetzung erfolgt.

Für das erfindungsgemäße Verfahren kann weiter beispielsweise vorgesehen sein, dass die erste bekannte Vergleichszusammensetzung (siehe Fig. 1, Schritt 15) eine oder mehrere der Zusammensetzungen aus Fig. 3A ist. Das Eisenoxidrotpigment wäre damit die dritte Komponente und das Dispergiermittel 1 die andere der beiden Komponenten und die zweite bekannte Vergleichszusammensetzung (siehe Fig. 1, Schritt 165) eine oder mehrere der Zusammensetzungen aus Fig. 3C ist. Das Eisenoxidrotpigment wäre damit die dritte Komponente und das Dispergiermittel 3 die Austauschkomponente.

Wie aus Figur 3C ersichtlich, ist für die Zusammensetzungen mit einem Dispergiermittelanteil von etwa 13 % die Farbstärke in ihrem Maximum M3, hier bei etwa 100 %, wobei bei einem Dispergiermittelanteil von etwa 15 bis 23 % die Viskosität [mPas] in ihrem Minimum ist. Da zumindest das Maximum M1 und M2 der Farbstärke aus den Figuren 3A und 3B (eher) weit von dem Maximum der Farbstärke M3 für Fig. 3C voneinander beabstandet ist (der Abstand ist etwa 5 % Dispergiermittelkonzentration), kann vorgesehen sein, dass die erste (Fig. 3A) und die zweite (Fig. 3C) bekannte Vergleichszusammensetzung als nicht ausreichend ähnlich bewertet werden. Hierbei ist ebenfalls aus den Fig. 3A und 3C ersichtlich, dass die Schnittpunkte der Lagerungszeitraumverläufe, zumindest für die Farbstärke, im Vergleich ebenfalls (eher) weit voneinander beabstandet sind. Somit können diese Schnittpunkte ebenfalls als Abstand in das Ähnlichkeitsmaß eingehen, wodurch in diesem Fall vorgesehen sein kann, dass die erste (Fig. 3A) und die zweite (Fig. 3C) bekannte Vergleichszusammensetzung nicht als ausreichend ähnlich bewertet werden.

### Bezugszeichenliste

- 10-19: Schritte
- 007: Benutzer
- 100: Computersystem
- 101: Interface
- 102: Prozessor
- 103: Arbeitsspeicher
- 200: Datenbank
- 500: Anlage zur Durchführung eines Herstellungsprozesses und Prüfung einer Formulierung für Farben, Lacke, Druckfarben, Anreibeharze, Pigmentkonzentrate oder sonstige Beschichtungsstoffe (HTE-Anlage)
- 506: Analysegerät
- 508: Analysegerät
- 510: Mischer
- 512: Syntheseeinheit
- 514: Syntheseeinheit
- 520: Prozessor
- M1: Maximum der Farbstärke für die Zusammensetzung in Fig. 3A
- M2: Maximum der Farbstärke für die Zusammensetzung in Fig. 3B
- M3: Maximum der Farbstärke für die Zusammensetzung in Fig. 3C
- A1: Abstand zwischen M1 und M2 bei Vergleich der der Figuren 3A und 3B
- A2: Abstand zwischen M2 und M3 bei Vergleich der der Figuren 3B und 3C

## Patentansprüche

1. Verfahren zur Generierung einer Zusammensetzung für Farben, Lacke, Druckfarben, Anreibeharze, Pigmentkonzentrate oder sonstige Beschichtungsstoffe, wobei die Zusammensetzung für Farben, Lacke, Druckfarben, Anreibeharze, Pigmentkonzentrate oder sonstige Beschichtungsstoffe Feststoffe und Dispergiermittel umfasst, wobei die Zusammensetzung durch ein Computersystem (100) generiert wird, wobei das Computersystem (100) auf eine Datenbank (200) Zugriff hat, in der bekannte Zusammensetzungen gespeichert sind und in der für Kombinationen eines Feststoffes und eines Dispergiermittels bekannter Zusammensetzungen jeweils Messpunkte der betreffenden Zusammensetzung für einen Feststoffanteil und eine Dispergiermittelkonzentration gespeichert sind, wobei für jeden Messpunkt der betreffenden Zusammensetzung eine rheologische und eine koloristische Eigenschaft gespeichert sind,
mit den Schritten:
a. Eingabe (10) einer Spezifikation einer gewünschten Kombination einer ersten Komponente und einer zweiten Komponente einer Zusammensetzung, wobei es sich bei den Komponenten um einen Feststoff und ein Dispergiermittel handelt, über ein Nutzerinterface (101) des Computersystems (100),
b. Durchführung (11) einer Datenbankabfrage mit der gewünschten Kombination der ersten Komponente und der zweiten Komponente als Suchkriterium,
(1) wobei für den Fall, dass die Datenbankabfrage eine bekannte Zusammensetzung liefert, die das Suchkriterium erfüllt, die bekannte Zusammensetzung ausgegeben wird (13),
(2) wobei im gegenteiligen Fall eine weitere Datenbankabfrage (14) einer Zusammensetzung mit der ersten Komponente und der zweiten Komponente als Suchkriterium durchgeführt wird, um eine Kandidatenzusammensetzung aufzufinden, die eine der beiden Komponenten sowie anstelle der anderen Komponente eine Austauschkomponente umfasst, wobei
i. eine Datenbankabfrage (15) durchgeführt wird, um eine erste bekannte Vergleichszusammensetzung aufzufinden, die die andere der beiden Komponente umfasst sowie eine dritte Komponente,
ii. eine Datenbankabfrage (16) durchgeführt wird, um eine zweite bekannte Vergleichszusammensetzung aufzufinden, die anstelle der anderen Komponente eine Austauschkomponente umfasst sowie die dritte Komponente,
iii. Vergleich (17) der ersten und der zweiten bekannten Vergleichszusammensetzung durch Prüfung eines Ähnlichkeitskriteriums hinsichtlich der in der Datenbank gespeicherten Messpunkte der ersten und der zweiten bekannten Vergleichszusammensetzung, wobei für den Fall, dass die erste und die zweite bekannte Vergleichszusammensetzung als ausreichend ähnlich bewertet werden, die
iv. Generierung (19) der Zusammensetzung durchgeführt wird, wobei in der Kandidatenzusammensetzung die Austauschkomponente gegen die andere der beiden Komponenten ausgetauscht wird, und die
v. Ausgabe (13) der generierten Zusammensetzung durchgeführt wird.

2. Verfahren gemäß Anspruch 1, wobei in dem Schritt b.(2) mehrere Kandidatenzusammensetzungen aufgefunden werden und die Schritte i. bis iii. so oft wiederholt werden bis für wenigstens eine der mehreren Kandidatenzusammensetzungen die Erfüllung des Ähnlichkeitskriteriums festgestellt wird und der Schritt iv. für diese Kandidatenzusammensetzung durchgeführt wird.

3. Verfahren gemäß Anspruch 1 oder Anspruch 2, wobei in dem Schritt b.(2) mehrere Kandidatenzusammensetzungen aufgefunden werden und die Schritte i. bis iii. mehrfach wiederholt werden bis für mehrere Kandidatenzusammensetzungen die Erfüllung des Ähnlichkeitskriteriums festgestellt wird, mit dem weiteren Schritt der Sortierung der Kandidatenzusammensetzungen, wobei die Kandidatenzusammensetzungen mit einem Ähnlichkeitsmaß als Sortierungskriterium sortiert werden, wobei die Kandidatenzusammensetzung mit dem höchsten Ähnlichkeitsmaß ausgewählt wird und der Schritt iv. für diese Kandidatenzusammensetzung durchgeführt wird.

4. Verfahren gemäß einem der Ansprüche 1 bis 3, wobei in das Ähnlichkeitsmaß ein erster Abstand der Extremwerte der rheologischen Eigenschaft und ein zweiter Abstand (A1; A2) der Extremwerte (M1; M2; M3) der koloristischen Eigenschaft, jeweils gegenüber einer Dispergiermittelkonzentration und einem Feststoffanteil, zwischen der ersten und der zweiten bekannten Vergleichszusammensetzung eingeht.

5. Verfahren gemäß Anspruch 4, wobei für die in der Datenbank gespeicherten Formulierungen Messpunkte unterschiedlicher Lagerungszeiträume gespeichert sind, wobei Schritt b.(2)iii. für Messpunkte verschiedener Lagerungszeiträume durchgeführt wird, um für jeden der Lagerungszeiträume die ersten und zweiten Abstände zu ermitteln.

6. Verfahren gemäß Anspruch 4 oder Anspruch 5, wobei sich das Ähnlichkeitsmaß aus einer Kombination der ersten und zweiten Abstände der Extremwerte ergibt.

7. Verfahren gemäß Anspruch 5 oder Anspruch 6, mit dem weiteren Schritt der Eingabe einer Spezifikation zumindest einer der Dimensionen der Messpunkte, wobei die ausgewählte Dimension in der Kombination übergewichtet wird.

8. Verfahren gemäß einem der vorherigen Ansprüche, mit dem weiteren Schritt der Eingabe der Spezifikation für einen Wertebereich der Messpunkte, wobei in Schritt b.(2)iii. die Messpunkte nur in dem spezifizierten Wertebereich ausgewertet werden.

9. Verfahren gemäß einem der Ansprüche 1 bis 3, wobei das Ähnlichkeitsmaß dem Vergleich der Verläufe der Messpunkte entspricht, wobei der Vergleich durch die Methode der kleinsten Quadrate, der Quadrate der Differenzen, eine Varianzanalyse, einen Stringvergleich von Messpunktbereichen oder eine Korrelation durchgeführt wird.

10. Verfahren gemäß einem der Ansprüche 1 bis 9, wobei
- in Schritt b.(2) i. die erste bekannte Vergleichszusammensetzung neben der anderen der beiden Komponenten und der dritten Komponente weitere Komponenten umfasst,
- in Schritt b.(2) ii. die zweite bekannte Vergleichszusammensetzung neben der dritten Komponenten und der Austauschkomponente ein, mehrere oder alle der weiteren Komponenten umfasst.

11. Verfahren gemäß einem der Ansprüche 1 bis 10, wobei die weiteren Komponenten ausgewählt sind aus der Gruppe bestehend aus Pigmenten, Füllstoffen, Farbstoffen, optischen Aufhellern, keramischen Materialien, magnetischen Materialien, nanodispersen Feststoffen, Metallen, Bioziden, Agrochemikalien, Pharmaka, Bindemitteln, Lösemitteln, Netzmitteln, Verlaufshilfsmitteln, Härtern und Entschäumern.

12. Verfahren gemäß einem der Ansprüche 1 bis 11, wobei der Feststoff ein anorganisches Pigment, ein organisches Pigment oder ein Rußpigment ist.

13. Verfahren gemäß einem der Ansprüche 1 bis 12, wobei das Dispergiermittel eine chemisch amphiphile, ionische, nichtionische oder niedermolekulare Verbindung und/oder hochmolekulare Verbindung ist.

14. Verfahren gemäß einem der Ansprüche 1 bis 13, wobei
- für den Fall, dass die eine der beiden Komponenten der Kandidatenzusammensetzung der Feststoff ist, der Feststoff ein anorganisches Pigment, ein organisches Pigment oder ein Rußpigment ist, die dritte Komponente entsprechend ebenfalls ein anorganisches Pigment, ein organisches Pigment oder ein Rußpigment ist, und
- für den Fall, dass die eine der beiden Komponenten der Kandidatenzusammensetzung das Dispergiermittel ist, das Dispergiermittel eine einfache, niedermolekulare Verbindung oder eine komplexe hochmolekulare Verbindung ist, die dritte Komponente entsprechend ebenfalls eine einfache, niedermolekulare Verbindung oder eine komplexe hochmolekulare Verbindung ist.

15. Verfahren gemäß einem der Ansprüche 1 bis 14, wobei die in der Datenbank (200) gespeicherten Messpunkte der ersten und der zweiten bekannten Vergleichszusammensetzung durch eine vorangegangene Prüfung der rheologischen und der koloristischen Eigenschaft gegenüber einer Dispergiermittelkonzentration und einem Feststoffanteil mittels einer Anlage (500) zur Durchführung eines Herstellungsprozesses einer Formulierung für Farben, Lacke, Druckfarben, Anreibeharze, Pigmentkonzentrate oder sonstige Beschichtungsstoffe und Prüfung einer Formulierung für Farben, Lacke, Druckfarben, Anreibeharze, Pigmentkonzentrate oder sonstige Beschichtungsstoffe gewonnen wurden.

16. Verfahren gemäß einem der Ansprüche 1 bis 15, wobei das Computersystem (100) über eine Kommunikationsschnittstelle mit der Datenbank (200) und/oder einer Anlage (500) zur Durchführung eines Herstellungsprozesses einer Formulierung für Farben, Lacke, Druckfarben, Anreibeharze, Pigmentkonzentrate oder sonstige Beschichtungsstoffe und Prüfung einer Formulierung für Farben, Lacke, Druckfarben, Anreibeharze, Pigmentkonzentrate oder sonstige Beschichtungsstoffe kommuniziert, wobei die Kommunikationsschnittstelle durch USB, Ethernet, WLAN, LAN, Bluetooth oder eine andere Netzwerkschnittstelle realisiert ist.

17. Verfahren gemäß einem der Ansprüche 1 bis 16, wobei nach Schritt b.(1) oder nach Schritt b.(2) v. die Ausgabe der generierten Zusammensetzung auf dem Nutzerinterface (101) des Computersystems (100) erfolgt.

18. Verfahren gemäß einem der Ansprüche 1 bis 17, wobei nach Schritt b.(1) oder nach Schritt b.(2) v. die Ausgabe der generierten Zusammensetzung an einen Prozessor (102) erfolgt, wobei der Prozessor (102) eine Anlage (500) zur Durchführung eines Herstellungsprozesses einer Formulierung für Farben, Lacke, Druckfarben, Anreibeharze, Pigmentkonzentrate oder sonstige Beschichtungsstoffe und Prüfung einer Formulierung für Farben, Lacke, Druckfarben, Anreibeharze, Pigmentkonzentrate oder sonstige Beschichtungsstoffe steuert, wobei die Anlage (500) mindestens zwei Bearbeitungsstationen aufweist, wobei die mindestens zwei Bearbeitungsstationen über ein Transportsystem miteinander verbunden sind, auf dem selbstfahrende Transportvehikel zum Transport der Komponenten der Formulierung und/oder der hergestellten Formulierung zwischen den Bearbeitungsstationen verkehren können, wobei der Prozessor (102) die Anlage (500) ansteuert, die generierte Zusammensetzung herzustellen, wobei in den mindestens zwei Bearbeitungsstationen die Herstellung der generierten Zusammensetzung und eine Prüfung der rheologischen und der koloristischen Eigenschaft gegenüber einer Dispergiermittelkonzentration und einem Feststoffanteil der Formulierung erfolgt, wonach eine Ausgabe (13) der Ergebnisse der Prüfung der rheologischen und der koloristischen Eigenschaft gegenüber der Dispergiermittelkonzentration und dem Feststoffanteil der Formulierung auf dem Nutzerinterface (101) erfolgt und/oder die Ergebnisse der Prüfung der rheologischen und der koloristischen Eigenschaft gegenüber der Dispergiermittelkonzentration und dem Feststoffanteil der Formulierung in der Datenbank (200) gespeichert werden.

19. Computersystem (100) zur Generierung einer Zusammensetzung für Farben, Lacke, Druckfarben, Anreibeharze, Pigmentkonzentrate oder sonstige Beschichtungsstoffe, umfassend eine Datenbank (200) und ein Nutzerinterface (101), wobei das Computersystem (100) zur Durchführung eines Verfahrens nach einem der Ansprüche 1 bis 18 konfiguriert ist.

20. Computerprogramm, digitales Speichermedium oder
Computerprogrammprodukt mit von einem Prozessor (102) ausführbaren Anweisungen, um ein Verfahren nach einem der Ansprüche 1 bis 18 durchzuführen.

21. System umfassend
- eine Anlage (500) zur Durchführung eines Herstellungsprozesses einer Formulierung für Farben, Lacke, Druckfarben, Anreibeharze, Pigmentkonzentrate oder sonstige Beschichtungsstoffe und Prüfung einer Formulierung für Farben, Lacke, Druckfarben, Anreibeharze, Pigmentkonzentrate oder sonstige Beschichtungsstoffe, wobei die Anlage (500) mindestens zwei Bearbeitungsstationen aufweist, wobei die mindestens zwei Bearbeitungsstationen über ein Transportsystem miteinander verbunden sind, auf dem selbstfahrende Transportvehikel zum Transport der Komponenten der Formulierung und/oder der hergestellten Formulierung zwischen den Bearbeitungsstationen verkehren können, wobei in den mindestens zwei Bearbeitungsstationen sowohl die Herstellung der Formulierung als auch eine Prüfung einer rheologischen Eigenschaft und einer koloristischen Eigenschaft gegenüber einer Dispergiermittelkonzentration und einem Feststoffanteil einer jeden Formulierung erfolgt, und
- ein Computersystem gemäß Anspruch 19.

## Claims

1. Method for generating a composition for paints, varnishes, printing inks, grinding resins, pigment concentrates or other coating materials, where the composition for paints, varnishes, printing inks, grinding resins, pigment concentrates or other coating materials comprises solids and dispersants, where the composition is generated by a computer system (100), where the computer system (100) has access to a database (200) in which known compositions are stored and in which, for combinations of a solid and a dispersant of known compositions, measurement points of the composition in question are stored in each case for a solids fraction and a dispersant concentration, where for each measurement point of the composition in question, a rheological property and a colouristic property are stored,
comprising the following steps:
a. input (10) of a specification of a desired combination of a first component and a second component of a composition, where the components are a solid and a dispersant, via a user interface (101) of the computer system (100),
b. implementation (11) of a database search with the desired combination of the first component and the second component as search criterion,
(1) where, if the database search yields a known composition which meets the search criterion, the known composition is output (13),
(2) where, in the opposite case, a further database search (14) for a composition having the first component and the second component as search criterion is implemented in order to discover a candidate composition which comprises one of the two components and, instead of the other component, a substitute component, where
i. a database search (15) is carried out in order to discover a first known comparative composition which comprises the other of the two components and also a third component,
ii. a database search (16) is carried out in order to discover a second known comparative composition which comprises, instead of the other component, a substitute component and also the third component,
iii. the first and second known comparative compositions are compared (17) by testing a similarity criterion in respect of the measurement points, stored in the database, of the first and second known comparative compositions, and, if the first and second known comparative compositions are rated as sufficiently similar,
iv. the composition is generated (19) with substitution in the candidate composition of the substitute component for the other of the two components, and
v. the generated composition is output (13).

2. Method according to Claim 1, wherein a plurality of candidate compositions are discovered in step b.(2) and steps i. to iii. are repeated until the similarity criterion is met for at least one of the plurality of candidate compositions, and step iv. is carried out for this candidate composition.

3. Method according to Claim 1 or Claim 2, wherein a plurality of candidate compositions are discovered in step b.(2) and steps i. to iii. are repeated multiply until the similarity criterion is met for a plurality of candidate compositions, with the further step of the sorting of the candidate compositions, where the candidate compositions are sorted with a degree of similarity as sorting criterion, where the candidate composition having the highest degree of similarity is selected and step iv. is carried out for this candidate composition.

4. Method according to any of Claims 1 to 3, wherein the degree of similarity incorporates a first distance between the extreme values of the rheological property and a second distance (A1; A2) between the extreme values (M1; M2; M3) of the colouristic property, in each case in relation to a dispersant concentration and a solids fraction, between the first and second known comparative compositions.

5. Method according to Claim 4, wherein measurement points of different storage periods are stored for the formulations stored in the database, where step b.(2)iii. is carried out for measurement points of different storage periods in order to ascertain the first and second distances for each of the storage periods.

6. Method according to Claim 4 or Claim 5, wherein the degree of similarity is obtained from a combination of the first and second distances between the extreme values.

7. Method according to Claim 5 or Claim 6, with the further step of input of a specification of at least one of the dimensions of the measurement points, where the selected dimension is overweighted in the combination.

8. Method according to any of the preceding claims, with the further step of input of the specification for a value range of the measurement points, where in step b.(2)iii. the measurement points are evaluated only in the specified value range.

9. Method according to any of Claims 1 to 3, wherein the degree of similarity corresponds to the comparison of the profiles of the measurement points, where the comparison is carried out by the method of least squares, of squares of the differences, a variance analysis, a string comparison of measurement point ranges or a correlation.

10. Method according to any of Claims 1 to 9, where
- in step b.(2) i. the first known comparative composition comprises further components as well as the other of the two components and the third component,
- in step b.(2) ii. the second known comparative composition comprises one, two or more or all of the further components as well as the third component and the substitute component.

11. Method according to any of Claims 1 to 10, wherein the further components are selected from the group consisting of pigments, fillers, dyes, optical brighteners, ceramic materials, magnetic materials, nanodisperse solids, metals, biocides, agrochemicals, drugs, binders, solvents, wetting agents, flow control assistants, curing agents and defoamers.

12. Method according to any of Claims 1 to 11, wherein the solid is an inorganic pigment, an organic pigment or a carbon black pigment.

13. Method according to any of Claims 1 to 12, wherein the dispersant is a chemically amphiphilic, ionic, nonionic or low molecular mass compound and/or high molecular mass compound.

14. Method according to any of Claims 1 to 13, where
- if the one of the two components of the candidate composition is the solid, the solid is an inorganic pigment, an organic pigment or a carbon black pigment and the third component, correspondingly, is likewise an inorganic pigment, an organic pigment or a carbon black pigment, and
- if the one of the two components of the candidate composition is the dispersant, the dispersant is a simple, low molecular mass compound or a complex, high molecular mass compound and the third component, correspondingly, is likewise a simple, low molecular mass compound or a complex, high molecular mass compound.

15. Method according to any of Claims 1 to 14, wherein the measurement points of the first and second known comparative compositions, stored in the database (200), have been obtained by a preceding test of the rheological property and the colouristic property in relation to a dispersant concentration and to a solids fraction by means of a facility (500) for implementing a process for producing a formulation for paints, varnishes, printing inks, grinding resins, pigment concentrates or other coating materials and testing a formulation for paints, varnishes, printing inks, grinding resins, pigment concentrates or other coating materials.

16. Method according to any of Claims 1 to 15, wherein the computer system (100) communicates via a communications interface with the database (200) and/or with a facility (500) for implementing a process for producing a formulation for paints, varnishes, printing inks, grinding resins, pigment concentrates or other coating materials and testing a formulation for paints, varnishes, printing inks, grinding resins, pigment concentrates or other coating materials, where the communications interface is realized by USB, Ethernet, WLAN, LAN, Bluetooth or another network interface.

17. Method according to any of Claims 1 to 16, wherein, after step b.(1) or after step b.(2) v., the generated composition is output on the user interface (101) of the computer system (100).

18. Method according to any of Claims 1 to 17, wherein, after step b.(1) or after step b.(2) v., the generated composition is output to a processor (102), where the processor (102) controls a facility (500) for implementing a process for producing a formulation for paints, varnishes, printing inks, grinding resins, pigment concentrates or other coating materials and testing a formulation for paints, varnishes, printing inks, grinding resins, pigment concentrates or other coating materials, where the facility (500) has at least two workstations, where the at least two workstations are connected to one another via a transport system on which self-propelled transport vehicles are able to run to transport the components of the formulation and/or of the formulation produced between the workstations, where the processor (102) drives the facility (500) to produce the generated composition, where the generated composition is produced and the rheological and colouristic properties are tested in relation to a dispersant concentration and a solids fraction of the formulation in the at least two workstations, after which the results of the test of the rheological and the colouristic properties in relation to the dispersant concentration and the solids fraction of the formulation are output (13) on the user interface (101) and/or the results of the test of the rheological and colouristic properties in relation to the dispersant concentration and the solids fraction of the formulation are stored in the database (200).

19. Computer system (100) for generating a composition for paints, varnishes, printing inks, grinding resins, pigment concentrates or other coating materials, comprising a database (200) and a user interface (101), where the computer system (100) is configured for implementing a method according to any of Claims 1 to 18.

20. Computer program, digital storage medium or computer program product having instructions which can be executed by a processor (102), to implement a method according to any of Claims 1 to 18.

21. System comprising
- a facility (500) for implementing a process for producing a formulation for paints, varnishes, printing inks, grinding resins, pigment concentrates or other coating materials and testing a formulation for paints, varnishes, printing inks, grinding resins, pigment concentrates or other coating materials, where the facility (500) has at least two workstations, where the at least two workstations are connected to one another via a transport system on which self-propelled transport vehicles are able to run in order to transport the components of the formulation and/or of the formulation produced between the workstations, where both the production of the formulation and testing of a rheological property and of a colouristic property in relation to a dispersant concentration and a solids fraction of each formulation take place in the at least two workstations, and
- a computer system according to Claim 19.

## Revendications

1. Procédé de génération d'une composition pour peintures, vernis, encres d'imprimerie, résines de transfert, concentrés de pigments ou autres matières de revêtement, la composition pour peintures, vernis, encres d'imprimerie, résines de transfert, concentrés de pigments ou autres matières de revêtement comprenant des matières solides et des agents dispersants, la composition étant générée par un système informatique (100), le système informatique (100) ayant accès à une base de données (200) dans laquelle sont mémorisées des compositions connues et dans laquelle sont mémorisées, pour des combinaisons d'une matière solide et d'un agent dispersant de compositions connues, des points de mesure de la composition respective pour une teneur en matière solide et une concentration d'agent dispersant, une propriété rhéologique et une propriété coloristique étant mémorisées pour chaque point de mesure de la composition concernée, le procédé comprenant les étapes suivantes :
a. entrer (10) une spécification d'une combinaison souhaitée d'un premier composant et d'un deuxième composant d'une composition, les composants étant une matière solide et un agent dispersant, par le biais d'une interface utilisateur (101) du système informatique (100),
b. effectuer (11) une requête auprès de la base de données avec la combinaison souhaitée du premier composant et du deuxième composant comme critère de recherche,
(1) si la requête auprès de la base de données délivre une composition connue satisfaisant au critère de recherche, la composition connue étant alors délivrée (13),
(2) dans le cas contraire, une nouvelle requête (14) auprès de la base de données étant effectuée concernant une composition ayant le premier composant et le deuxième composant comme critère de recherche afin de trouver une composition candidate qui comprend l'un des deux composants et un composant de remplacement à la place de l'autre composant,
i. une requête (15) auprès de la base de données étant effectuée afin de trouver une première composition de comparaison connue qui comprend l'autre des deux composants et un troisième composant,
ii. une requête (16) auprès de la base de données étant effectuée afin de trouver une deuxième composition de comparaison connue qui comprend un composant de remplacement à la place de l'autre composant et le troisième composant,
iii. la comparaison (17) de la première et de la deuxième composition de comparaison connue par vérification d'un critère de similarité concernant les points de mesure de la première et de la deuxième composition de comparaison connue qui sont mémorisés dans la base de données, si les première et deuxième compositions de comparaison connues sont évaluées comme suffisamment similaires, alors
iv. la génération (19) de la composition est effectuée, le composant de remplacement remplaçant l'autre des deux composants dans la composition candidate, et
v. la délivrance (13) de la composition générée étant effectuée.

2. Procédé selon la revendication 1, une pluralité de compositions candidates étant trouvées à l'étape b.(2) et étant répétées aux étapes i. à iii. jusqu'à ce que la satisfaction au critère de similarité soit déterminée pour l'une au moins de la pluralité de compositions candidates et l'étape iv. est réalisée pour cette composition candidate.

3. Procédé selon la revendication 1 ou la revendication 2, une pluralité de compositions candidates étant trouvées à l'étape b.(2) et répétées plusieurs fois aux étapes i. à iii. jusqu'à ce que la satisfaction au critère de similarité soit déterminée pour plusieurs compositions candidates, avec l'étape supplémentaire de tri des compositions candidates, les compositions candidates étant triées à l'aide d'une mesure de similarité comme critère de tri, la composition candidate ayant le degré de similarité le plus élevé étant sélectionnée et l'étape iv. étant réalisée pour cette composition candidate.

4. Procédé selon l'une des revendications 1 à 3, le degré de similarité impliquant une première distance entre les valeurs extrêmes de la propriété rhéologique et une deuxième distance (A1 ; A2) entre les valeurs extrêmes (M1 ; M2 ; M3) de la propriété coloristique, à chaque fois par rapport à une concentration d'agent dispersant et à une teneur en matière solide, entre la première et la deuxième composition de comparaison connue.

5. Procédé selon la revendication 4, des points de mesure de différentes durées de stockage étant mémorisés pour les formulations mémorisées dans la base de données, l'étape b. (2)iii. étant effectuée pour mesurer des points de durées de stockage différentes afin de déterminer les première et deuxième distances pour chacune des durées de stockage.

6. Procédé selon la revendication 4 ou la revendication 5, le degré de similarité résultant d'une combinaison des première et deuxième distances entre les valeurs extrêmes.

7. Procédé selon la revendication 5 ou la revendication 6, comprenant en outre l'étape d'entrée d'une spécification d'au moins une des dimensions des points de mesure, la dimension sélectionnée étant surpondérée dans la combinaison.

8. Procédé selon l'une des revendications précédentes, comprenant l'étape supplémentaire d'entrée de la spécification pour une plage de valeurs des points de mesure, les points de mesure n'étant évalués à l'étape b.(2)iii. que dans la plage de mesure spécifiée.

9. Procédé selon l'une des revendications 1 à 3, le degré de similarité correspondant à la comparaison des variations des points de mesure, la comparaison étant effectuée par la méthode des moindres carrés, les carrés des différences, une analyse de variance, une comparaison en chaîne de plages de points de mesure ou une corrélation.

10. Procédé selon l'une des revendications 1 à 9,
- à l'étape b.(2)i. la première composition de comparaison connue comprenant d'autres composants en plus de l'autre des deux composants et du troisième composant,
- à l'étape b.(2)ii. la deuxième composition de comparaison connue comprenant, en plus du troisième composant et du composant de remplacement, un autre composant, plusieurs autres composants ou tous les autres composants.

11. Procédé selon l'une des revendications 1 à 10, les autres composants étant choisis dans le groupe comprenant les pigments, les charges, les colorants, les azurants optiques, les matériaux céramiques, les matériaux magnétiques, les matières solides nano-dispersées, les métaux, les biocides, les produits agrochimiques, les produits pharmaceutiques, les liants, les solvants, les agents mouillants, les auxiliaires d'égalisation, les durcisseurs et les agents anti-moussants.

12. Procédé selon l'une des revendications 1 à 11, la matière solide étant un pigment minéral, un pigment organique ou un pigment de noir de carbone.

13. Procédé selon l'une des revendications 1 à 12, l'agent dispersant étant un composé chimiquement amphiphile, ionique, non ionique ou de faible poids moléculaire et/ou un composé de poids moléculaire élevé.

14. Procédé selon l'une des revendications 1 à 13,
- si l'un des deux composants de la composition candidate est la matière solide, la matière solide étant alors un pigment minéral, un pigment organique ou un pigment de noir de carbone, le troisième composant étant également un pigment minéral, un pigment organique ou un pigment de noir de carbone, et
- si l'un des deux composants de la composition candidate est le dispersant, le dispersant étant alors un composé simple de faible poids moléculaire ou un composé complexe de poids moléculaire élevé, le troisième composant étant également un composé simple de faible poids moléculaire ou un composé complexe de poids moléculaire élevé.

15. Procédé selon l'une des revendications 1 à 14, les points de mesure des première et deuxième compositions de comparaison connues, qui sont mémorisés dans la base de données (200), ayant été obtenus par une vérification préalable des propriétés rhéologiques et coloristiques par rapport à une concentration d'agent dispersant et une teneur en matière solide au moyen d'une installation (500) destinée à effectuer un processus de production d'une formulation pour peintures, vernis, encres d'imprimerie, résines de transfert, concentrés de pigments ou autres matières de revêtement et par une vérification d'une formulation pour peintures, vernis, encres d'imprimerie, résines de transfert, concentrés de pigments ou autres matières de revêtement.

16. Procédé selon l'une des revendications 1 à 15, le système informatique (100) communiquant par le biais d'une interface de communication avec la base de données (200) et/ou une installation (500) destinée à réaliser un processus de production d'une formulation pour peintures, vernis, encres d'imprimerie, résines de transfert, concentrés de pigments ou autres matières de revêtement et à vérifier une formulation pour peintures, vernis, encres d'imprimerie, résines de transfert, concentrés de pigments ou autres matières de revêtement, l'interface de communication étant réalisée par USB, Ethernet, WLAN, LAN, Bluetooth ou une autre interface réseau.

17. Procédé selon l'une des revendications 1 à 16, la délivrance de la composition générée étant effectuée après l'étape b.(1) ou après l'étape b.(2)v. sur l'interface utilisateur (101) du système informatique (100) .

18. Procédé selon l'une des revendications 1 à 17, la composition générée étant délivrée à un processeur (102) après l'étape b. (1) ou après l'étape b.(2)v., le processeur (102) commandant une installation (500) destinée à réaliser un processus de production d'une formulation pour peintures, vernis, encres d'imprimerie, résines de transfert, concentrés de pigments ou autres matières de revêtement et vérifier une formulation pour peintures, vernis, encres d'imprimerie, résines de transfert, concentrés de pigments ou autres matières de revêtement, l'installation (500) comportant au moins deux postes de traitement, les au moins deux postes de traitement étant reliés l'un à l'autre par le biais d'un système de transport sur lequel des véhicules de transport automoteurs destinés à transporter les composants de la formulation et/ou la formulation produite peuvent circuler entre les postes de traitement, le processeur (102) commandant l'installation (500) pour produire la composition générée, la production de la composition générée et une vérification des propriétés rhéologiques et coloristiques par rapport à une concentration d'agent dispersant et une teneur en matière solide de la formulation étant effectuées dans les au moins deux postes de traitement, après quoi une délivrance (13) des résultats de la vérification des propriétés rhéologiques et coloristiques par rapport à la concentration d'agent dispersant et à la teneur en matière solide de la formulation étant effectuée sur l'interface utilisateur (101) et/ou les résultats de la vérification des propriétés rhéologiques et coloristiques par rapport à la concentration d'agent dispersant et de la teneur en matière solide de la formulation étant mémorisés dans la base de données (200).

19. Système informatique (100) destiné à générer une composition pour peintures, vernis, encres d'imprimerie, résines de transfert, concentrés de pigments ou autres matières de revêtement, ledit système informatique comprenant une base de données (200) et une interface utilisateur (101), le système informatique (100) étant conçu pour exécuter un procédé selon l'une des revendications 1 à 18.

20. Programme d'ordinateur, support de mémorisation numérique ou progiciel comprenant des instructions exécutables par un processeur (102) pour mettre en oeuvre un procédé selon l'une des revendications 1 à 18.

21. Système comprenant
- une installation (500) destinée à exécuter un procédé de production d'une formulation pour peintures, vernis, encres d'imprimerie, résines de transfert, concentrés de pigments ou autres matières de revêtement et vérifier une formulation pour peintures, vernis, encres d'imprimerie, résines de transfert, concentrés de pigments ou autres matière de revêtement, l'installation (500) comprenant au moins deux postes de traitement, les au moins deux postes de traitement étant reliés l'un à l'autre par le biais d'un système de transport sur lequel des véhicules de transport automoteurs destinés à transporter les composants de la formulation et/ou la formulation produite peuvent circuler entre les postes de traitement, la production de la formulation ainsi qu'une vérification d'une propriété rhéologique et d'une propriété coloristique par rapport à une concentration d'agent dispersant et une teneur en matière solide de chaque formulation étant effectuées dans les au moins deux postes de traitement, et
- un système informatique selon la revendication 19.
